(19) Europäisches Patentamt
European Patent Office
Office européen des brevets

(11) Numéro de publication: **0 102 909**
**A1**

(12) # DEMANDE DE BREVET EUROPEEN

(21) Numéro de dépôt: 83420132.9

(51) Int. Cl.[3]: C 07 D 249/12

(22) Date de dépôt: 21.07.83

(30) Priorité: 06.08.82 FR 8214028

(43) Date de publication de la demande: 14.03.84 Bulletin 84/11

(84) Etats contractants désignés: AT BE CH DE FR GB IT LI LU NL SE

(71) Demandeur: RHONE-POULENC AGROCHIMIE
14-20, rue Pierre Baizet
F-69009 Lyon(FR)

(72) Inventeur: Michelet, Daniel
Les Erables No. 21 24, Chemin de Montribloud
F-69160 Tassin de la demi lune(FR)

(72) Inventeur: Veracini, Serge
36, Avenue de Ménival
F-69005 Lyon(FR)

(74) Mandataire: Brachotte, Charles et al,
RHONE-POULENC AGROCHIMIE P.I.D. BP 9163
F-69263 Lyon Cedex 09(FR)

(54) **Procédé de préparation d'hydroxy-triazoles utilisables comme intermédiaires pour les herbicides.**

(57) Procédé de préparation d'hydroxy-3 triazoles-1,2,4, utilisables comme intermédiaires pour faire des herbicides, qui consiste à faire réagir une semicarbazide avec un sel d'iminium à groupe méthylène substitué dissymétriquement.

EP 0 102 909 A1

Croydon Printing Company Ltd

La présente invention concerne un procédé de préparation d'intermédiaires pour la synthèse d'esters phosphoriques de triazoles-1,2,4.

On connait divers esters phosphoriques de triazoles-1,2,4 (brevets français 1 567 668, 2 156 865, 2 156 864, 2 162 592) ainsi que leurs intermédiaires et les procédés de préparation de ces intermédiaires.

Les procédés connus de préparations d'esters phosphoriques de triazoles 1,2,4, partent généralement de réactifs contenant le cycle triazolyle déja formé, par exemple des hydroxy-3 triazoles 1,2,4 de formule :

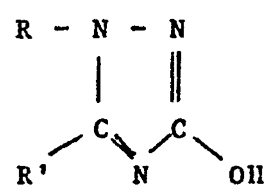

(I)

R et R' ayant des significations assez diverses.

On peut préparer les réactifs de formule (I) par réaction de semicarbazides avec l'acide formique (Ber. 26 p.2613 (1893)), ou par réaction de semicarbazides avec des orthocarboxylates d'alkyle, notamment des orthoformiates. On peut aussi faire réagir les semicarbazides avec le formaldéhyde, puis on cyclise ensuite en milieu alcalin, et, éventuellement, on soumet le produit obtenu à une halogénation.

Bien que certaines de ces réactions aient pu conduire à des procédés industrialisés, ces procédés ne sont pas satisfaisants ; en effet, les rendements sont généralement médiocres, aussi bien dans les réactions mises en oeuvre dans l'acide formique que dans les réactions effectuées en présence d'orthoformiates. Toutefois, dans ce dernier cas, il apparait possible d'atteindre d'assez bons rendements (J. Am. Chem. Soc. 77, 5872 (1955)), à condition d'engager de forts excès d'orthoformiates qui sont des

BAD ORIGINAL

réactifs chers. Quoiqu'il en soit, les procédés actuels conduisent à des esters phosphoriques de triazole tellement coûteux que celà constitue même un frein à leur développement.

Un but de la présente invention est de fournir un procédé de préparation d'hydroxy-3 triazole-1,2,4 ou de ses dérivés n'ayant pas les inconvénients des procédés connus.

Le procédé selon l'invention est caractérisé en ce qu'on fait réagir une semicarbazide avec des sels d'iminium, ceux-ci pouvant éventuellement être générés in situ. Le procédé de l'invention permet donc de préparer des produits de formule (I), R et R' étant le plus souvent l'atome d'hydrogène ou radicaux organiques.

Comme semicarbazide utilisable, on peut citer les semicarbazides de formule :

$$R-NH-NH-CO-NH_2$$

dans laquelle R représente :

+ un atome d'hydrogène, ou

+ un radical alkyle, alkényle ou phényle, éventuellement substitué, notamment par :

un ou plusieurs atomes d'halogène, notamment de chlore, de brome et/ou d'iode, des radicaux alkyle inférieurs, éventuellement halogénés tels que $CF_3$, des radicaux alcoxy inférieurs, alkylthio inférieurs, alkylsulfinyles inférieurs, alkylsulfonyle inférieurs ou nitro, ou,

+ un radical phénylalkyle ou diphénylalkyle (à partie alkyle inférieure), non substitué ou substitué, par exemple par un atome d'halogène ou un radical alkyle ou alcoxy.

D'une manière générale on préfère mettre en oeuvre les réactifs dans lesquels R comporte 1 à 6 atomes de carbone, ou 6 à 18 atomes de carbone si R contient un noyau phényle.

Dans le présent exposé, on désigne par l'adjectif "inférieur" les radicaux ayant de 1 à 6 atomes de carbone et de préférence de 1 à 4 atomes de carbone.

Comme sels d'iminium, on utilise généralement des sels d'iminium à groupe méthylène substitué dissymétriquement. Plus spécialement on peut utiliser les sels de formule

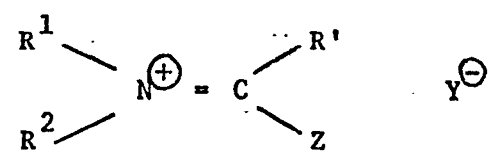 (II)

dans laquelle $R^1$ et $R^2$, identiques ou différents, représentent un radical alkyle ayant de préférence de 1 à 5 atomes de carbone, ou encore un radical divalent de formule

$$-(CH_2)_n (X)_{n'} - (CH_2)_n- \qquad (III)$$

dans laquelle

X est un groupe méthylène ou un hétéroatome tel que l'atome d'oxygène ou d'azote

n est un nombre entier de 2 à 6, de préférence égal à 2

n' est un nombre entier égal à 0 ou 1

R' est un atome d'hydrogène ou un groupement alkyle, alkényle, aryle ou aralkyle.

Z est un atome d'halogène ou un groupement de

formule

$$-A(B)_{n''}$$

A étant un hétéroatome électronégatif tel que O ou N, et

B un radical divalent de formule (III), ou un groupement alkyle, aryle, arylsulfonyle, ou alkylarylsulfonyle

R' et Z comprennent de préférence au plus 6 atomes de carbone, ou au plus 12 atomes de carbone s'ils comportent un noyau phényle

n" est un nombre entier égal à 1 ou 2

Z est de préférence un atome de chlore, un groupement alkyloxy, dialkylamino, notamment diméthylamino, pipéridyle, sulfonyloxy.

Y est tel que YH soit un acide fort minéral ou organique, tel l'acide chlorhydrique, un monoester de polyacide tel que les acides sulfurique ou sulfoniques, ou bien un alcool, ou bien une amine secondaire.

Les sels d'iminium de formule (II) peuvent être préparés par tout moyen connu en soi. On peut, par exemple, faire réagir du phosgène, un sulfate d'alkyle ou un chlorure d'acide arylsulfonique sur du diméthylformamide (DMF) ou l'un de ses homologues.

Selon une variante du procédé, les sels d'iminium de formule (II) sont formés in situ par dissociation de précurseurs tels que les orthoamides de formule (IV), suivant la réaction équilibrée :

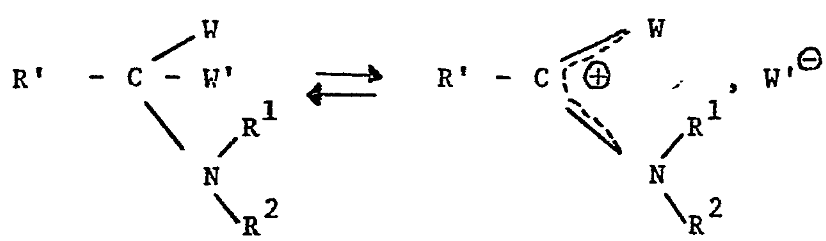

(IV)

R', $R^1$, $R^2$ ayant les significations précédemment indiquées et W et W' étant des groupements alkyloxy ou dialkylamino.

Comme orthoamides, on utilise de préférence le diméthyl acétal du diméthylformamide ( R' = H ; $R_1 = R_2 = CH_3$ ; W = W' = $OCH_3$) les alkoxy bis (dialkylamino) méthane (R' = H ; W = N($R'_1$) $R'_2$ ; W' = $OR_3$ ; $R_1$, $R'_1$, $R_2$, $R'_2$, $R_3$ = alkyl), ou les tris (dialkylamino) méthane (R' = H ; W = N($R'_1$)$R'_2$ ; W' = N($R"_1$)$R"_2$ ; $R_1$, $R'_1$, $R"_1$, $R_2$, $R'_2$, $R"_2$ = alkyl) et les composés similaires, notamment ceux à groupes cycliques, deux radicaux alkyle formant ensemble un radical unique divalent.

Le procédé selon l'invention est généralement mis en oeuvre entre 0 et 200°C. Le choix de la température réactionnelle dépend des réactifs mis en oeuvre. Ainsi lorsque Z et Y sont l'atome de chlore, on opère généralement par mélange des réactifs à basse température (entre 5 à 50°C par exemple, de préférence entre 15 à 35°C), puis par chauffage entre 80 et 180°C.

Dans le cas où Y est tel que YH est un sulfate d'alkyle, un alcool, ou une amine secondaire, on peut se contenter de mettre en contact les réactifs entre 5 et 50°C.

La réaction est généralement effectuée dans un milieu solvant. Comme solvant utilisable on peut citer : les nitriles, notamment l'acétonitrile ; les amides, notamment le diméthylformamide ; les hydrocarbures aromatiques substitués ou non, tels que le nitrobenzène, le chlorobenzène, l'anisole ; les hydrocarbures aliphatiques halogénés tels que le dichloroéthane ; les esters tels que l'acétate d'éthyle ; les carbonates tels que le carbonate de méthyle.

Le sel d'iminium, lorsqu'il est liquide, ou son précurseur, peuvent éventuellement servir eux-mêmes de solvant.

Comme solvant, on utilise de préférence l'acétonitrile, ou bien le diméthylformamide dans le cas où R' est l'atome d'hydrogène.

Les exemples suivants, donnés à titre non limitatif, illustrent l'invention et montrent comment elle peut être mise en oeuvre.

Les rendements indiqués sont exprimés par rapport à la semicarbazide engagée.

Exemple 1 :

Dans un réacteur A de 100 ml muni d'une agitation et placé sous atmosphère inerte, on charge 2,20g (22,2 millimoles) de phosgène dans 10 ml d'acétonitrile. La température du mélange est abaissée à -20°C. A l'aide d'une ampoule de coulée, on introduit en 30 minutes 1,42g (19,4 millimoles) de diméthylformamide en solution dans 15 ml d'acétonitrile. On ajoute 10 ml d'acétonitrile provenant du rinçage de l'ampoule de coulée. On élimine sous pression réduite (20 mm Hg) le dioxyde de carbone formé et le phosgène excédentaire tandis qu'on laisse remonter la température à 0°C.

Dans un réacteur B de 100 ml muni d'une agitation et placé sous atmosphère inerte, on charge 2,92g (19,3 millimoles) de phénylsemicarbazide dans 15 ml d'acétonitrile. Sur la suspension obtenue, on charge rapidement (cinq minutes) à température ambiante le contenu du réacteur A. Il se forme un léger précipité de coloration rose. On laisse I h 30 à température ambiante sous agitation, puis distille le solvant sous pression réduite jusqu'à obtention de 4,736g d'un solide blanc.

Ce solide est chargé sous atmosphère inerte dans un

autoclave avec 40 ml d'acétonitrile. L'autoclave est chauffé pendant 2 heures à 130°C. Après filtration, on obtient 2,84g d'un solide cristallisé titrant 100 % en phényl-1 hydroxy-3 triazole-1, 2, 4 (mesuré par potentiométrie). Point de fusion : 282°C.

Rendement = 91,5 %.

Exemple 2 :

On opère comme dans l'exemple 1 jusqu'à obtention de 4,70g du solide blanc intermédiaire. Ce solide est chauffé en masse à 170°C pendant une heure. Après lavage à l'eau bouillante, filtration et lavage à température ambiante avec de l'eau distillée, on obtient 2,83g d'un solide titrant 100 % en triazole. Point de fusion : 281°C.

Rendement : 91 %.

Exemple 3 :

Dans un réacteur A de 100 ml muni d'une agitation et placé sous atmosphère inerte, on charge 25 ml de diméthylformamide (DMF) sur lequel on coule, à -30°C, 1,85g (18,6 millimoles) de phosgène. Il se forme un précipité blanc en suspension dans le DMF.

Cette suspension est chargée dans un réacteur B de 100 ml également muni d'une agitation et placé sous atmosphère inerte, contenant 2,80g (18,5 millimoles) de phénylsemicarbazide en suspension dans 10 ml de DMF. Cette opération est effectuée à température ambiante ; on ajoute 15 ml de DMF provenant du rinçage du réacteur A. On laisse sous agitation pendant 20 minutes à température ambiante, jusqu'à obtention d'une solution homogène de coloration jaune.

Cette solution est chauffée au reflux du DMF (153°C) pendant 2 heures. La masse réactionnelle prend une coloration rouge. Le solvant est distillé sous pression

réduite ; le résidu est lavé par 60 ml d'eau distillée. On récupère 2,86 g d'un solide titrant 97 % (17,2 millimoles) en phényl-1 hydroxy-3 triazole-1,2,4,. Point de fusion : 281°C..

Rendement : 93 %.

Exemple 4 :

Dans un réacteur A de 100 ml muni d'une agitation et placé sous atmosphère inerte, on charge 3,76 g (29,8 millimoles) de sulfate de méthyle. On introduit 2,20 g (30,1 millimoles) de diméthylformamide et on porte la masse réactionnelle à 80°C pendant 3 h 30 minutes. Après refroidissement, on obtient une solution légèrement colorée en jaune.

Dans un réacteur B de 100 ml muni d'une agitation et placé sous atmosphère inerte, on charge 4,50 g (29,8 millimoles) de phénylsemicarbazide en suspension dans 25 ml d'acétonitrile. Sur cette suspension on coule à température ambiante la solution préparée dans le réacteur A et 15 ml d'acétonitrile. On ajoute 10 ml d'acétonitrile provenant du rinçage du réacteur A, puis on chauffe la masse réactionnelle à 50°C pendant 3 heures sous agitation. On distille alors l'acétonitrile jusqu'à obtention de 9,68 g d'un résidu qui est ensuite lavé par 30 ml d'eau distillée. On récupère 1,72 g d'un solide blanc titrant 99,4 % (10,6 millimoles) en phényl-1 hydroxy-3 triazole-1,2,4. Point de fusion : 281°C. Dans la phase aqueuse, on dose 0,42 g (2,6 millimoles) supplémentaire de triazole.

Rendement : 44 %.

Exemple 5 :

On opère comme dans l'exemple précédent à partir de 2,51 g (19,9 millimoles) de sulfate de méthyle, 1,47 g (20,2 millimoles) de diméthylformamide, et 3,00 g (19,8 millimoles) de phénylsemicarbazide.

Lorsque l'addition est terminée, on laisse pendant 30 heures à température ambiante sous agitation. Le solvant est distillé et le résidu lavé avec 20 ml d'eau distillée. On récupère 1,54 g d'un solide titrant 64 % (6,1 millimoles) en triazole. La phase aqueuse contient 0,22 g (1,4 millimole) supplémentaire de triazole.

Rendement : 38 %.

Exemple 6 :

Dans un réacteur de 100 ml muni d'une agitation et placé sous atmosphère inerte, on charge 3,01 g (19,9 millimoles) de phénylsemicarbazide dans 25 ml d'acétonitrile. On coule sous agitation, en 20 minutes à température ambiante, une solution de 3,23 g (18,6 millimoles) de tertio-butoxy-bis (diméthylamino) méthane (BBDM) dans 15 ml d'acétonitrile. La masse réactionnelle prend immédiatement une coloration rose.

Lorsque la coulée est terminée, on laisse sous agitation pendant 2 h 30 supplémentaires à température ambiante, puis on laisse reposer une nuit. Le solvant est ensuite distillé pour obtenir 3,88 g d'un résidu qu'on lave avec 20 ml d'eau distillée. On acidifie le milieu jusqu'à pH compris entre 3 et 4 pour précipiter 2,35 g d'un solide titrant 98,5 % (14,4 millimoles) en phényl-1 hydroxy-3 triazole-1,2,4. Point de fusion : 279°C.

Rendement : 72 %.

Exemple 7 :

Dans un réacteur de 100 ml muni d'une agitation et placé sous atmosphère inerte, on charge 1,91g (12,6 millimoles) de phénylsemicarbazide en suspension dans 25 ml d'acétonitrile. On coule sous agitation, en 15 minutes à température ambiante, une suspension de 3,35g (12,6 millimoles) de trispipéridino méthane (TPM) dans 20 ml d'acétonitrile. La masse réactionnelle prend une coloration rose dès le début de la coulée.

On ajoute 10 ml d'acétonitrile provenant du lavage de l'ampoule de coulée, et on laisse sous agitation pendant 5 heures à température ambiante. Le solvant est ensuite distillé pour obtenir 3,19g d'un résidu qu'on lave avec 80 ml d'eau distillée. Le milieu est acidifié jusqu'à pH compris entre 3 et 4 pour obtenir 1,88g d'un solide titrant 99,7 % en phényl-1 hydroxy-3 triazole-1,2,4. Point de fusion : 281°C.

Rendement = 92 %.

Exemple 8 :

Dans un réacteur A de 100 ml muni d'une agitation et placé sous atmosphère inerte, on charge 3,35g (22,2 millimoles) de phénylsemicarbazide en suspension dans 25 ml d'acétonitrile.

Dans une ampoule de coulée B, on charge dans 10 ml d'acétonitrile 4,98g d'un réactif dont la composition est la suivante :

- Ethoxy bis (diméthylamino) méthane (EBDM) 32,6 % (en poids) ; 11,1 millimoles.
- Tris (diméthylamino) méthane (TDM) : 12,1 % (en poids) ; 4,1 millimoles
- Diéthylacétal du diméthylformamide : 19,2 % (en poids) ; 6,5 millimoles
- Diméthylformamide : 36,1 % (en poids).

Le contenu de l'ampoule B est coulé en 15 minutes dans le réacteur A maintenu sous agitation à température ambiante. On ajoute 15 ml d'acétonitrile provenant du lavage de l'ampoule B et on laisse pendant 4 heures à température ambiante sous agitation. Le solvant est ensuite distillé pour obtenir 5,10g d'un résidu qu'on lave avec 50 ml

d'eau distillée. Le milieu est acidifié jusqu'à pH compris entre 3 et 4 pour obtenir 2,92g d'un solide titrant 97,6 % en phényl-1 hydroxy-3 triazole 1,2,4. Point de fusion : 281°C.

Rendement = 80 %.

Exemple 9 :

Dans un réacteur A de 100 ml muni d'une agitation et placé sous atmosphère inerte, on charge 4,03 g de chlorure de p. toluènesulfonyle titrant 94 % (19,9 millimoles) et 1,45 g (19,8 millimoles) de diméthylformamide dans 10 ml d'acétonitrile. Ce mélange réactionnel est chauffé à reflux pendant 1 heure. On obtient une solution homogène qu'on laisse ensuite reposer à température ambiante pendant une nuit.

Dans un réacteur B de 100 ml muni d'une agitation et placé sous atmosphère inerte, on charge 2,99 g (19,8 millimoles) de phénylsemicarbazide dans 25 ml d'acétonitrile. Sur la suspension obtenue, on coule en 15 minutes à température ambiante, la solution contenue dans le réacteur A. On ajoute encore 15 ml d'acétonitrile provenant du lavage du réacteur A et on laisse le mélange réactionnel, initialement homogène, pendant 4 heures à température ambiante, puis pendant 3 jours à 50°C sous agitation. A l'issue de cette opération, le mélange réactionnel est devenu hétérogène, avec apparition d'un précipité blanc. Le solvant est distillé sous pression réduite, le résidu est lavé avec 50 ml d'eau distillée jusqu'à obtention de 2,56 g d'un solide blanc titrant 96,6 % (15,3 millimoles) en phényl-1 hydroxy-3 triazole-1,2,4. Point de fusion : 279°C.

Rendement = 77 %.

REVENDICATIONS

1) Procédé de préparation d'hydroxy-3 triazoles-1,2,4, caractérisé en ce qu'on fait réagir une semicarbazide avec un sel d'iminium à groupe méthylène substitué dissymétriquement.

2) Procédé selon la revendication 1, caractérisé en ce que le sel d'iminium a pour formule :

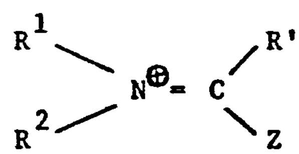 , $Y^{\ominus}$ (II)

et la semicarbazide a pour formule :

$$R - NH - NH - CO - NH_2$$

dans lesquelles R et R' sont l'atome d'hydrogène ou des radicaux organiques.

3) Procédé selon la revendication 2 caractérisé en ce que

- R représente un atome d'hydrogène ou un radical alkyle, ou alkényle, ou phényle, ou phénylalkyle ou diphénylalkyle, éventuellement substitué
- R' est un atome d'hydrogène ou un groupement alkyle, ou alkényle, ou aryle, ou aralkyle
- $R^1$ et $R^2$, identiques ou différents, représentent des radicaux alkyle, ou forment ensemble un radical divalent de formule :

$$-(CH_2)_n -(X)_{n'} -(CH_2)_n- \quad (III)$$

- Z est un atome d'halogène ou un groupement de formule : $-A(B)_{n''}$
- X est un groupe méthylène ou un hétéroatome tel que O ou N
- n est un nombre entier de 2 à 6, de préférence égal à 2
- n' est égal à 0 ou 1

- A est un hétéroatome électronégatif, de préférence O ou N
- B est un radical divalent de formule (III) ou un groupe alkyle ou aryle ou arylsulfonyle ou alkylarylsulfonyle
- n" est égal à 1 ou 2
- Y est tel que YH est un acide fort minéral ou organique, ou un monoester de polyacide, ou un alcool, ou une amine secondaire

4) Procédé selon l'une des revendications 1 à 3, caractérisé en ce que lorsque $R^1$ et $R^2$ représentent un groupe alkyle, ils ont au plus 5 atomes de carbone, et/ou que Z est choisi dans le groupe constitué par le chlore, ou un radical alkyloxy, dialkylamino, diméthylamino, pipéridyle, sulfonyloxy.

5) Procédé selon l'une des revendications 1 à 4, caractérisé en ce que le sel d'iminium est formé in situ à partir d'un précurseur du type orthoamide.

6) Procédé selon l'une des revendications 1 à 5, caractérisé en ce que la réaction est effectuée entre 0 et 200°C éventuellement en présence d'un solvant.

7) Procédé selon la revendication 6, caractérisé en ce que le solvant est un nitrile ou un amide ou un hydrocarbure aromatique éventuellement substitué, ou un hydrocarbure aliphatique halogéné, ou un carbonate.

8) Procédé selon l'une des revendications 6 ou 7, caractérisé en ce que Z et Y sont l'atome de chlore et que la réaction est effectuée d'abord entre 5 et 50°C, et ensuite entre 80 et 180°C.

9) Procédé selon l'une des revendications 6 ou 7, caractérisé en ce que Y est tel que YH est un sulfate d'alkyle, un alcool, ou une amine secondaire et que la réaction est effectuée entre 5 et 35°C.

10) Procédé selon l'une des revendications 1 à 9, caractérisé en ce que R est le radical phényle, R' est l'atome d'hydrogène.

0102909

Office européen des brevets

# RAPPORT DE RECHERCHE EUROPEENNE

Numéro de la demande

EP 83 42 0132

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | BULLETIN DES SOCIETES CHIMIQUES BELGES, vol. 84, no. 10, juillet 1975, pages 1005-1012 G. GERMAIN et al.: "Synthèse et alnalyse cristallographique par diffraction de Rayons X d'un sel de triazolium formé à partir de nouveaux réactifs bis-électrophiles 1,4, dérivant de la condensation des sels de phosgène iminium et des hyxdrazines monosubstituées" * L'article pages 1005-1012 * | 1 | C 07 D 249/12 |
| | --- | | |
| A | EP-A-0 062 853 (HOECHST) * Revendications * | 1 | |
| | --- | | |
| A | GB-A-2 077 718 (CIBA-GEIGY) * En entier * | 1 | DOMAINES TECHNIQUES RECHERCHES (Int. Cl. 3) |
| | ----- | | C 07 D 249/00 |

Le présent rapport de recherche a été établi pour toutes les revendications

| Lieu de la recherche | Date d'achèvement de la recherche | Examinateur |
|---|---|---|
| LA HAYE | 07-11-1983 | CREMERS K. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503.03.82